(19) Europäisches Patentamt
European Patent Office
Office européen des brevets

(11) Veröffentlichungsnummer: **0 305 593**
**A2**

(12) # EUROPÄISCHE PATENTANMELDUNG

(21) Anmeldenummer: **87117526.1**

(51) Int. Cl.4: **C07D 213/64 , C07D 213/73**

(22) Anmeldetag: **30.11.84**

(30) Priorität: **06.12.83 CH 6509/83**
**18.04.84 CH 1948/84**

(43) Veröffentlichungstag der Anmeldung:
**08.03.89 Patentblatt 89/10**

(60) Veröffentlichungsnummer der früheren Anmeldung nach Art. 76 EPÜ: **0 148 119**

(84) Benannte Vertragsstaaten:
**BE CH DE FR GB IT LI NL SE**

(71) Anmelder: **CIBA-GEIGY AG**
**Klybeckstrasse 141**
**CH-4002 Basel(CH)**

(72) Erfinder: **Böhner, Beat, Dr.**
**Hügelweg 3**
**CH-4102 Binningen(CH)**
Erfinder: **Rempfler, Hermann, Dr.**
**Brücklismattstrasse 16**
**CH-4107 Ettingen(CH)**
Erfinder: **Schurter, Rolf, Dr.**
**Holzmattstrasse 45**
**CH-4102 Binningen(CH)**

(54) **Neue Zwischenprodukte für 2-Pyridinyloxyphenoxypropionsäure-cyanamide.**

(57) 2-Pyridinyloxyphenoxypropionsäure-cyanamide der Formel

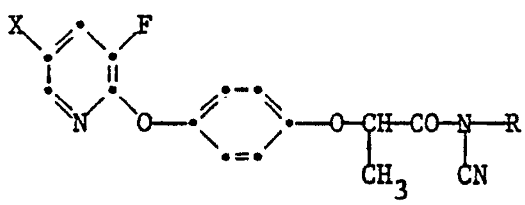

haben eine nützliche selektive Herbizidwirkung gegen Unkräuter, vorzugsweise gegen Schadgräser in Nutzpflanzenkulturen. In dieser Formel bedeuten
R Wasserstoff, $C_1$-$C_4$-Alkyl, $C_3$-$C_4$-Alkenyl, $C_3$-$C_4$-Alkinyl oder $C_2$-$C_4$-Alkoxyalkyl, und
X Fluor, Chlor, Brom, Jod oder Trifluormethyl.
Die bei der Herstellung durchlaufenen Zwischenprodukte der Formeln XIV, XV, XVIII und IIa sind neu:

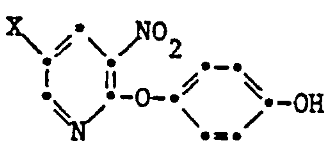

(XIV) ,

EP 0 305 593 A2

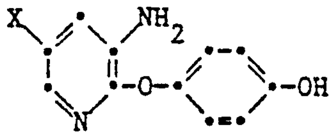

(XV) ,

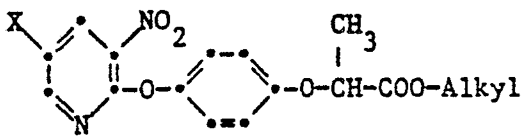

(XVIII) ,

worin Alkyl für $C_1$-$C_4$-Alkyl steht und

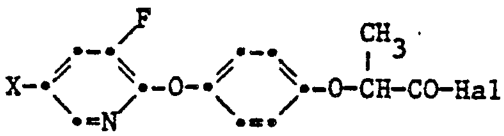

(IIa)

worin Hal für Chlor oder Brom und X für Fluor, Chlor, Brom oder Jod stehen.

## Neue Zwischenprodukte für 2-Pyridinyloxyphenoxypropionsäure-cyanamide

Die vorliegende Erfindung betrifft neue Zwischenprodukte für herbizid wirksame 2-Pyridinyloxyphenoxypropionsäure-cyanamide. Die Verwendung der wirksamen Endprodukte und der sie enthaltenden Mittel zur selektiven Bekämpfung von Unkräutern in Kulturpflanzenbeständen ist in unserer publizierten Stammanmeldung EP-A-148 119 beschrieben.

Die wirksamen 2-Pyridinyloxyphenoxypropionsäure-cyanamide entsprechen der Formel Ic

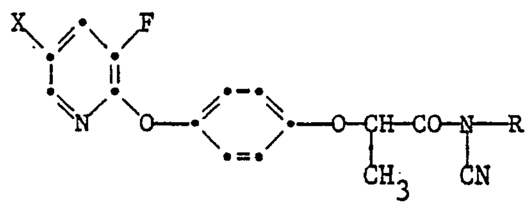

(Ic)

worin
R Wasserstoff, $C_1$-$C_4$Alkyl, $C_3$-$C_4$-Alkenyl, $C_3$-$C_4$-Alkinyl oder $C_2$-$C_4$-Alkoxyalkyl,
X Fluor, Chlor, Brom, Jod oder Trifluormethyl bedeuten.

Herbizide 2-Phenoxypropionsäure-Amide mit weiteren Substituenten in der para-Stellung des Phenylkerns sind aus der Literatur, wie beispielsweise den Deutschen Offenlegungsschriften 2 433 067, 2 531 643, 2 639 796, 2 640 730 oder 3 004 770 und der publizierten Europäischen Patentanmeldung EP-A-21453 bekannt.

Es wurde gefunden, dass die neuen Wirkstoffe der Formel Ic gemäss EP-A-148 119 den literaturbeschriebenen Verbindungen dieser Substanzklasse in der selektiven Unkrautbekämpfung in Nutzpflanzenkulturen überlegen sind.

Im Rahmen der EP-A-148 119 fallen unter das in der Definition der Formel I verwendete Symbol R beispielsweise folgende Substituenten: R steht im allgemeinen für Wasserstoff, Methyl, Aethyl, Isopropyl, n-Propyl, die vier isomeren Butyl, Allyl, Methallyl, 2-Butenyl, 3-Butenyl, Propargyl, 2-Butinyl, 3-Butinyl, Methoxymethyl, Methoxyäthyl, Aethoxymethyl sowie Aethoxyäthyl.

Als bevorzugte Wirkstoffe werden genannt:
2-[4-(5-Chlor-3-fluorpyridin-2-yloxy)-phenoxy]-propionsäure-N-cyan-N-methyl-amid und
2-[4-(5-Chlor-3-fluorpyridin-2-yloxy)-phenoxy]-propionsäure-N-cyan-N-äthyl-amid.

Ueber ein neues Verfahren kann man Verbindungen der Formel Ic

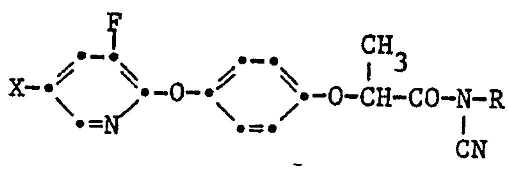

(Ic) herstellen,

indem man entweder

a) ein 3-Nitropyridin der Formel XIII

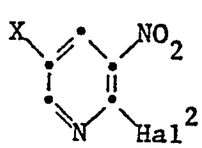

(XIII)

worin X die unter Formel Ic gegebene Bedeutung hat und $Hal^2$ für Fluor, Chlor oder Brom steht, in Gegenwart einer Base mit Hydrochinon zu einer Verbindung der Formel XIV

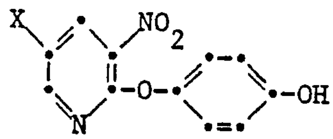

(XIV)

umsetzt, dieses Zwischenprodukt in Gegenwart eines Metallkatalysators zu einer Aminoverbindung der Formel XV

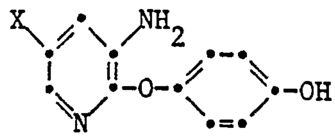

(XV)

reduziert, diese diazotiert und mit einem Fluorierungsmittel in eine Verbindung der Formel XVI

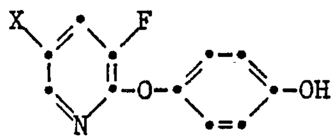

(XVI)

überführt und dieses fluorierte Zwischenprodukt in Gegenwart einer Base mit einem Propionsäure-Derivat der Formel VII

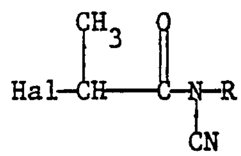

(VII)

worin R die unter Formel Ic gegebene Bedeutung hat und Hal für Chlor, Brom, Tosyl oder Mesyl steht, umsetzt; oder indem man

b) das Nitropyridin der Formel (XIII) in Gegenwart einer Base mit einem Propionsäure der Formel XVII

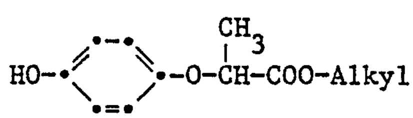

(XVII)

worin Alkyl für $C_1$-$C_4$-Alkyl steht, umsetzt, das erhaltene Zwischenprodukt der Formel XVIII

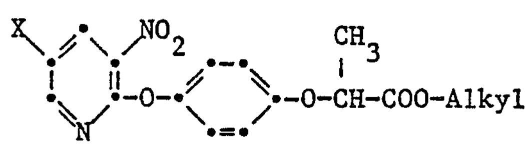

(XVIII)

in Gegenwart eines Metallkatalysators zur Aminoverbindung der Formel XIX

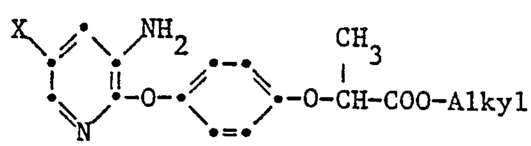

(XIX)

reduziert, dieses Zwischenprodukt diazotiert und mit einem Fluorierungsmittel in eine Verbindung der

Formel XX

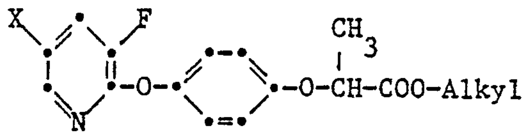
(XX)

überführt, diesen Ester verseift und mit einem Halogenierungsmittel in ein 2-Phenoxypropionsäurehalogenid der Unterformel IIa

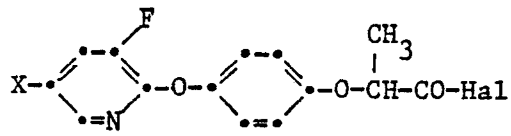
(IIa)

worin Hal für Chlor oder Brom und X für Fluor, Chlor, Brom, Jod oder Trifluormethyl stehen, überführt und in Gegenwart einer Base mit einem Cyanamin der Formel III

H— N —R (III)
 |
 CN

umsetzt.

Gemäss einer Variante des obigen Verfahrens kann man die Zwischenprodukte der Formel XVIII auch erhalten, indem man die Verbindungen der Formel XIV in Gegenwart einer Base mit einem Propionsäureester der Formel XXI

 $CH_3$
 |
Hal- CH -COO-Alkyl (XXI)

worin Hal für Chlor oder Brom und Alkyl für $C_1$-$C_4$-Alkyl stehen, umsetzt.

Die Reaktionsbedingungen für die an sich bekannten Reaktionsschritte, wie Katalysatoren, Lösungsmittel und Reaktionstemperaturen, sind aus der Literatur bekannt.

Die Ausgangsprodukte der Formeln XIII und XVII sind bekannt.

Die durchlaufenen Zwischenprodukte der Formeln XIV, XV und XVIII sind neu und wurden speziell für die Synthese der Verbindungen der Formel Ic entwickelt. Sie bilden die Erfindungsgegenstände der vorliegenden Erfindung. Ebenfalls neu sind die Verbindungen der Formel IIa, worin X für Halogen steht. Diese Verbindungen bilden einen weiteren Aspekt der vorliegenden Erfindung.

Die Verbindungen der Formel Ic fallen als Racemate an. Gegenstand der EP-A-148 119 sind beide Enantiomeren der Wirkstoffe der Formel Ic und Gemische davon. Sofern nicht ausdrücklich ein bestimmtes Isomeres erwähnt ist, beziehen sich die in der EP-A-148 119 gemachten Feststellungen stehts auf die Racemate.

Die Wirkstoffe der Formel Ic sind stabile Verbindungen. Ihre Handhabung bedarf keiner vorsorglichen Massnahmen.

Bei geringeren Aufwandmengen zeichnen sich die Verbindungen der Formel Ic durch gute selektiv-herbizide Eigenschaften, insbesondere gegen monokotyle Unkräuter aus, die sie ausgezeichnet zum Einsatz in Kulturen von dikotylen Nutzpflanzen, insbesondere Baumwolle, Soja, Raps, Zucker- und Futterrüben sowie Sonnenblumen, befähigen. Zum Teil können die erfindungsgemässen Verbindungen der Formel I aber auch als Selektivherbizide in monokotylen Kulturen wie Getreide, beispielsweise Weizen und Gerste, eingesetzt werden. Es werden dabei teilweise auch Unkräuter geschädigt, welchen bisher nur mit Totalherbiziden beizukommen war.

Bei höheren Aufwandmengen entfalten die Wirkstoffe der Formel Ic totalherbizide Eigenschaften.

Die selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung, insbesondere gegen Schadgräser, gleichermassen mit gutem Erfolg verwendet werden.

## Herstellungsbeispiele

Beispiel H1: 2-[4-(5-Chlor-3-fluorpyridin-2-ylxoy)-phenoxy]-propionsäure-N-cyan-N-methylamid

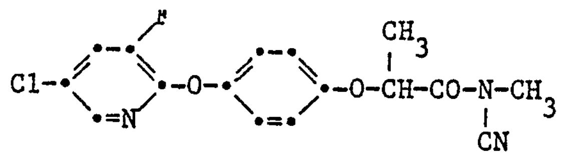

a) 4-(5-Chlor-3-nitropyridin-2-yloxy)-phenol.

Ein Gemisch aus 85,9 g (0,78 Mol) Hydrochinon, 1200 ml Acetonitril und 53,9 g (0,39 Mol) Kaliumcarbonat wird auf eine Temperatur von 60 °C erwärmt. Dann lässt man innerhalb von 6 Stunden eine Lösung von 115,8 g (0,60 Mol) 2,5-Dichlor-3-nitro-pyridin in 500 ml Acetonitril zutropfen und rührt die Mischung für weitere 36 Stunden. Das Lösungmittel wird abdestilliert und der Rückstand in ein Eiswasser/Salzsäure-Gemisch gegossen. Nach der Extraktion mit Methylenchlorid werden die vereinigten organischen Phasen über Magnesiumsulfat getrocknet, mit Aktivkohle behandelt, filtriert und eingeengt. Der Rückstand wird durch Kristallisation in Hexan/Aethylacetat-Gemisch gereinigt. Man erhält 99 g (62% d.Th.)4-(5-Chlor-3-nitropyridin-2-yloxy)-phenol, Smp. 125-126 °C.

b) 4-(3-Amino-5-chlorpyridin-2-yloxy)-phenol.

110,7 g (0,415 Mol) 4-(5-Chlor-3-nitropyridin-2-yloxy)-phenol werden in 1200 ml Dioxan gelöst und,nach Zugabe von 22,0g Raney-Nickel-Katalysator, bei 20-25 °C mit Wasserstoff hydriert. Dann wird der Katalysator abfiltriert, die Lösung eingeengt und der Rückstand mit Hexan verrührt. Nach dem Filtrieren und Trocknen werden 96,0 g (98% d.Th.) 4-(3-Amino-5-chlorpyridin-2-yloxy)-phenol erhalten, Smp. 174 °C.

c) 4-(5-Chlor-3-fluorpyridin-2-yloxy)-phenol.

In 400 g (20 Mol) vorgelegten Fluorwasserstoff trägt man bei einer Temperatur zwischen -8 °C und 0 °C 122,5 g (0,518 Mol) 4-(3-Amino-5-chlorpyridin-2-yloxy)-phenol ein. Dann werden innerhalb von einer Stunde portionsweise 37,3 g (0,540 Mol) Natriumnitrit eingetragen. Das Gemisch wird für 2 Stunden bei 0 °C gerührt und im Autoklaven langsam auf 55 °C erwärmt. Das überflüssige Fluorwasserstoff wird abdestilliert, der Rückstand mit 200 ml Methylenchlorid aufgenommen, mit Eiswasser und Ammoniak neutralisiert. Nach Trocknen und Eindampfen der organischen Phase erhält man 112 g 4-(5-Chlor-3-fluorpyridin-2-yloxy)-phenol, Smp. 97-98 °C.

d) 48,0 g (0,20 Mol) 4-(5-Chlor-3-fluorpyridin-2-yloxy)-phenol und 38,2 g (0,20 Mol) 2-Brompropionsäure-N-cyan-N-methylamid werden in 400 ml Acetonitril gelöst, mit 35,9 g (0,26 Mol) Kaliumcarbonat und 0,33 g (0,002 Mol) Kaliumjodid versetzt und für 13 Stunden auf 80 °C erhitzt. Der Niederschlag wird abgetrennt, das Filtrat eingedampft und der Rückstand in 500 ml Methylenchlorid aufgenommen. Zur Reinigung wird die Lösung mit Aktivkohle behandelt und durch Kieselgel filtriert. Das Filtrat wird eingedampft und der Rückstand aus Hexan/Aethylacetat-Gemisch kristallisiert, wodurch man 61 g 2-[4-(5-Chlor-3-fluorpyridin-2-yloxy)-phenoxy]-propionsäure-N-cyan-N-methylester erhält.

Beispiel H2: 2-[4-(5-Chlor-3-fluorpyridin-2-yloxy)-phenoxy]-propionsäure-N-n-butyl-N-cyanamid

a) 2-[4-(5-Chlor-3-nitropyridin-2-yloxy)-phenoxy]-propionsäuremethylester.

38,6 g (0,20 Mol) 2,5-Dichlor-3-nitropyridin, 41,2 g (0,21 Mol) 2-(4-Hydroxyphenoxy)-propionsäuremethylester, 400 ml Acetonitril, 35,9 g (0,26 Mol) Kaliumcarbonat und 0,33 g (0,002 Mol) Kaliumjodid werden für 13 Stunden auf eine Temperatur von 80 °C erhitzt. Das Reaktionsgemisch wird filtriert und der Filterkuchen mit Acetonitril nachgewaschen. Das Filtrat wird eingedampft und der Rückstand in 500 ml Methylenchlorid aufgenommen. Nach Behandlung der Lösung mit 15 g Aktivkohle wird sie durch eine Schicht Kieselgel filtriert und das Filtrat eingedampt. Der Rückstand wird aus Hexan/Aethylacetat-Gemisch kristallisiert. Man erhält 65,7 g (93,1% d.Th.) 2-[4-(5-Chlor-3-nitropyridin-2-yloxy)-phenoxy]-propionsäuremethylester, Smp. 91-92 °C.

b) 2-[4-(3-Amino-5-chlorpyridin-2-yloxy)-phenoxy]-propionsäuremethylester.

190,8 g (0,541 Mol) 2-[4-(5-Chlor-3-nitropyridin-2-yloxy)-phenoxy]-propionsäuremethylester werden in 1,8 lt Dioxan gelöst, mit 40 g Raney-Nickel-Katalysator versetzt und bei 20-25 °C mit Wasserstoff hydriert. Anschliessend wird der Katalysator abfiltriert, das Lösungsmittel abdestilliert und der noch warme ölige Rückstand unter Einrühren in Hexan kristallisiert. Der Niederschlag wird abgetrennt und getrocknet. Man erhält so 167,4 g (96% d.Th.) 2-[4-(3-Amino-5-chlorpyridin-2-yloxy)-phenoxy]-propionsäuremethylester, Smp. 92-94 °C.

c) 2-[4-(5-Chlor-3-fluorpyridin-2-yloxy)-phenoxy]-propionsäuremethylester.

400 g (20 Mol) Fluorwasserstoff werden vorgelegt und bei einer Temperatur zwischen -8° C und 0° C portionsweise mit 167,4 g (0,518 Mol) 2-[4-(3-Amino-5-chlorpyridin-2-yloxy)-phenoxy]-propionsäuremethylester versetzt. Im Verlaufe von 1 Stunde setzt man 37,3 g (0,540 Mol) Natriumnitrit zu und rührt die Mischung für weitere 2 Stunden, bevor man sie im Autoklaven langsam auf 55° C erhitzt. Anschliessend wird das überschüssige Fluorwasserstoff abdestilliert und der Rückstand in eine Mischung aus 200 ml Methylenchlorid und Eiswasser aufgenommen und mit konzentriertem Ammoniak neutralisiert. Durch dreimalige Extraktion mit Methylenchlorid, Waschen mit Wasser, Trocknen und Eindampfen der organischen Extrakte erhält man 81,0 g (48% d.Th.) 2-[4-(5-Chlor-3-fluorpyridin-2-yloxy)-phenoxy]-propionsäuremethylester, Smp. 63-64° C.

d) 2-[4-(5-Chlor-3-fluorpyridin-2-yloxy)-phenoxy]-propionsäure.

Ein Gemisch aus 67,0 g (0,206 Mol) 2-[4-(5-Chlor-3-fluorpyridin-2-yloxy)-phenoxy]-propionsäuremethylester, 350 ml Dioxan und 250 ml 1N Natriumhydroxid-Lösung wird für 2 Stunden bei einer Temperatur von 40° C gerührt. Das Reaktionsgemisch wird auf, eine Mischung aus Eisund 150 ml 2N Salzsäure gegossen und zweimal mit Aethylacetat extrahiert. Die Extrakte werden mit gesättigter Natriumchloridlösung gewaschen, über Magnesiumsulfat getrocknet, filtriert und eingedampft. Man erhält 63,6 g (99% d.Th.) 2-[4-(5-Chlor-3-fluorpyridin-2-yloxy)-phenoxy]-propionsäure, Smp. 95-97° C.

e) 2-[4-(5-Chlor-3-fluorpyridin-2-yloxy)-phenoxy]-propionylchlorid.

63,6 g 2-[4-(5-Chlor-3-fluorpyridin-yloxy)-phenoxy]-propionsäure werden in 700 ml Toluol gelöst. Aus dieser Lösung werden 200 ml Toluol abdestilliert. Nach dem Abkühlen auf 90° C setzt man tropfenweise 25 ml (0,34 Mol) Thionylchlorid zu und rührt die Lösung für weitere 14 Stunden bei dieser Temperatur. Durch Eingengen verringert man das Volumen auf 200 ml. Die erhaltene toluolische Lösung von 2-[4-(5-Chlor-3-fluorpyridin-2-yloxy)-phenoxy]-propionylchlorid wird direkt in die folgende Reaktionsstufe eingesetzt.

f) Zu einer Lösung von 3,5 g (0,035 Mol) Triäthylamin und 3,2 g (0,033 Mol) n-Butylcyanamin in 40 ml Toluol lässt man unter Eiskühlung eine Lösung von 9,9 g (0,030 Mol) 2-[4-(5-Chlor-3-fluorpyridin-2-yloxy)-phenoxy]-propionylchlorid in 30 ml Toluol zutropfen. Nach einer Reak tionszeit von 5 Stunden trennt man den Niederschlag ab und reinigt das Filtrat durch Chromatographie an Kieselgel. Durch Eindampfen des Eluats erhält man 2-[4-(5-Chlor-3-fluorpyridin-2-yloxy)-phenoxy]-propionsäure-N-n-butyl-N-cyanamid in Form eines gelbstichigen Oeles, $n_D^{25}$ : 1.5482.

<u>Beispiel H3</u>: 2R-[4-(5-Chlor-3-fluorpyridin-2-yloxy)-phenoxy]-propionsäure-N-äthyl-N-cyanamid.

Zu einer Lösung von 2,1 g (0,030 Mol) Aethylcyanamin und 9,9 g (0,030 Mol) 2R-[4-(5-Chlor-3-fluorpyridin-2-yloxy)-phenoxy]-propionylchlorid (Smp. 47-48° C) in 30 ml Acetonitril wird unter Eiskühlung eine Lösung von 4,43 g (0,030 Mol) 1,8-Diazabicyclo[5.4.0]undec-7-en (97 proz.) in 10 Acetonitril getropft. Nach einer Reaktionszeit von 4 Std. wird das Reaktionsgemisch eingeengt. Das gewünschte 2R-[4-(5-Chlor-3-fluorpyridin-2-yloxy)-phenoxy]-propionsäure-N-äthyl--N-cyanamid erhält man durch Chromatographie an Kieselgel und Eindampfen des Eluates (Lösungsmittelgemisch: Aethylacetat/Hexan 1:1).

In analoger Weise werden die in den folgenden Tabellen aufgelisteten Zwischenprodukte und Verbindungen der Formel I erhalten.

Tabelle 1:

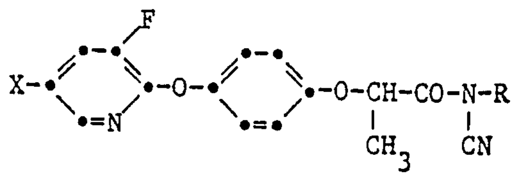

| Verb. Nr. | X | R | phys. Daten |
|---|---|---|---|
| 1.1 | Cl | H | |
| 1.2 | Cl | $CH_3$ | |
| 1.3 | Cl | $C_2H_5$ | $n_D^{25}$: 1.5545 |
| 1.4 | Cl | $C_4H_9$-n | $n_D^{25}$: 1.5482 |
| 1.5 | Cl | $-CH_2-CH=CH_2$ | |
| 1.6 | Cl | $-(CH_2)_2-OCH_3$ | |

Tabelle 2:

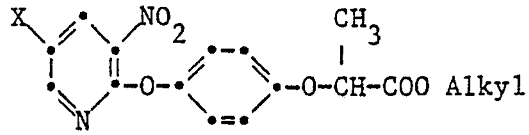

| Verb. Nr. | X | Alkyl | phys. Daten |
|---|---|---|---|
| 2.1 | Cl | $CH_3$ | Smp. 91-92°C |
| 2.2 | Br | $CH_3$ | |
| 2.3 | F | $CH_3$ | |
| 2.4 | Cl | $C_2H_5$ | |
| 2.5 | Cl | $C_4H_9$-n | |
| 2.6 | $CF_3$ | $CH_3$ | |
| 2.7 | $CF_3$ | $C_4H_9$-n | |

Tabelle 3:

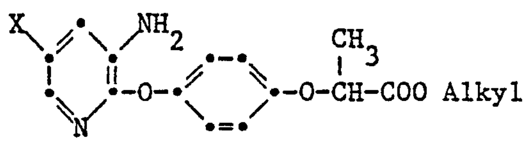

| Verb. Nr. | X | Alkyl | phys. Daten |
|---|---|---|---|
| 3.1 | Cl | $CH_3$ | Smp. 92-94°C |
| 3.2 | Br. | $CH_3$ | |
| 3.3 | F | $CH_3$ | |
| 3.4 | Cl | $C_2H_5$ | |
| 3.5 | Cl | $C_4H_9$-n | Oel |
| 3.6 | $CF_3$ | $CH_3$ | |
| 3.7 | $CF_3$ | $C_4H_9$-n | |

Tabelle 4:

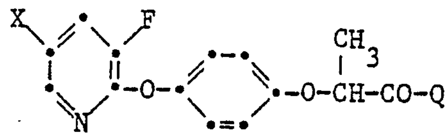

| Verb. Nr. | X | Q | phys. Daten |
|---|---|---|---|
| 4.1 | Cl | OH | Smp. 95-97°C |
| 4.2 | Cl | ONa | |
| 4.3 | Cl | OK | |
| 4.4 | Br | OH | |
| 4.5 | F | OH | |
| 4.6 | $CF_3$ | OH | |
| 4.7 | Cl | Cl | Oel |
| 4.8 | Cl | Br | |
| 4.9 | Cl | F | |
| 4.10 | Br | Cl | |
| 4.11 | F | Cl | |
| 4.12 | $CF_3$ | Cl | |

Tabelle 5:

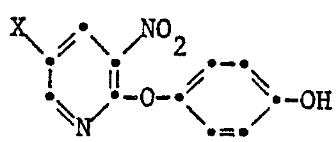

| Verb. Nr. | X | phys. Daten |
|---|---|---|
| 5.1 | Cl | Smp. 125-126°C |
| 5.2 | Br | |
| 5.3 | F | |
| 5.4 | $CF_3$ | |

Tabelle 6:

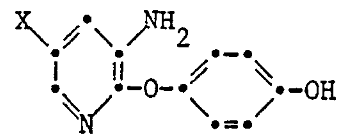

| Verb. Nr. | X | phys. Daten |
|---|---|---|
| 6.1 | Cl | Smp. 174°C |
| 6.2 | Br | |
| 6.3 | F | |
| 6.4 | $CF_3$ | |

Tabelle 7:

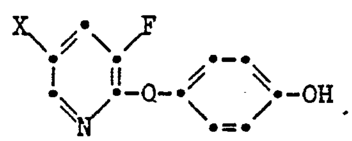

| Verb. Nr. | X | phys. Daten |
|---|---|---|
| 7.1 | Cl | Smp. 97-98°C |
| 7.2 | Br | |
| 7.3 | F | |
| 7.4 | $CF_3$ | |

## Ansprüche

1. Verbindungen der Formel XIV

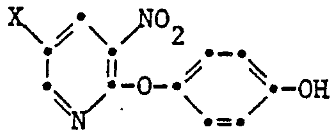 (XIV)

worin X Fluor, Chlor, Brom, Jod oder Trifluormethyl bedeutet.

2. 4-(5-Chlor-3-nitropyridin-2-yloxy)-phenol gemäss Anspruch 1.

3. Verbindungen der Formel XV

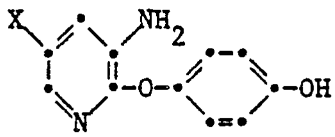 (XV)

worin X Fluor, Chlor, Brom, Jod oder Trifluormethyl bedeutet.

4. 4-(3-Amino-5-chlorpyridin-2-yloxy)-phenol gemäss Anspruch 3.

5. Verbindungen der Formel XVIII

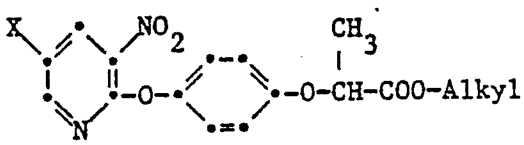 (XVIII)

worin X für Fluor, Chlor, Brom, Jod oder Trifluormethyl und Alkyl für $C_1$-$C_4$-Alkyl stehen.

6. 2-[4-(5-Chlor-3-nitropyridin-2-yloxy)-phenoxy]-propionsäuremethylester gemäss Anspruch 5.

7. Verbindungen der Formel IIa

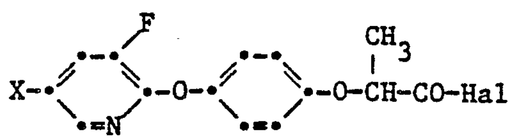 (IIa)

worin Hal für Chlor oder Brom und X für Fluor, Chlor, Brom oder Jod stehen.

8. 2-[4-(5-Chlor-3-fluorpyridin-2-yloxy)-phenoxy]-propionylchlorid gemäss Anspruch 7.